(19) Europäisches Patentamt
European Patent Office
Office européen des brevets

(11) Publication number: 0 123 327 B1

(12) EUROPEAN PATENT SPECIFICATION

(45) Date of publication of patent specification: 09.09.87

(51) Int. Cl.4: C 07 D 213/40, C 07 D 237/08, C 07 D 241/12, A 01 N 43/54, A 01 N 43/58, A 01 N 43/60, A 01 N 43/40, C 07 D 239/26, C 07 D 239/30, C 07 D 239/52

(21) Application number: 84200268.5

(22) Date of filing: 24.02.84

(54) **Chloroacetanilides, their preparation, their use, compositions containing them, and method of combating undesired plant growth.**

(30) Priority: 28.03.83 GB 8308474

(43) Date of publication of application: 31.10.84 Bulletin 84/44

(45) Publication of the grant of the patent: 09.09.87 Bulletin 87/37

(84) Designated Contracting States: AT BE CH DE FR GB IT LI LU NL SE

(58) References cited:
EP-A-0 024 017
DE-A-2 742 583

(73) Proprietor: SHELL INTERNATIONALE RESEARCH MAATSCHAPPIJ B.V.
Carel van Bylandtlaan 30
NL-2596 HR Den Haag (NL)

(72) Inventor: Munro, David
40 Harvesters Way
Maidstone Kent (GB)
Inventor: Ten Haken, Pieter
"Konkelboet" The Street
Eastling - nr. Faversham Kent (GB)
Inventor: Webb, Shirley Beatrice
17 North Street Ashford Road
Sheldwich Faversham Kent (GB)

(74) Representative: Hunter, Keith Roger Ian et al
4 York Road
London SE1 7NA (GB)

EP 0 123 327 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present application relates to chloroacetanilides, to a process for their preparation, to compositions containing them, to a method of combatting undesired plant growth using them, and to their use as herbicides.

Chloroacetanilides having herbicidal properties are known from the literature. Thus, 2-chloro-2′,6′-diethyl-N-(methoxymethyl)acetanilide, common name alachlor, is known to be active against a variety of weeds, although at the dosages required for combatting certain grasses, it can damage crops as well. Analogous compounds having N-heterocyclicmethyl substituents are disclosed in GB—A—1,585,243 and 1,588,874 and EP—A—0024017.

It has now been found that a specific group of novel chloroacetanilides show useful selective herbicidal activity. Accordingly, the present invention provides chloroacetanilide derivatives of the general formula I:—

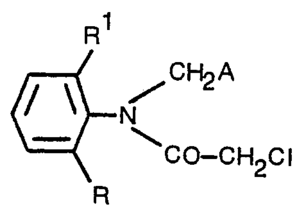

(I)

wherein A represents an optionally substituted azine selected for pyridyl, pyrazinyl, pyridazinyl, 4-pyrimidinyl and 5-pyrimidinyl, optional substituents being selected from $C_{1-4}$ alkyl, $C_{1-4}$ alkoxy, $C_{1-4}$ haloalkyl and hydroxy groups and halogen atoms; and R and $R^1$ which may be the same or different, each independently represents an alkyl group having up to four carbon atoms.

When the amine group A is substituted, preferred substituents are methyl, ethyl, methoxy, propoxy, trifluoromethyl, and chloromethyl groups and chloro and bromo atoms; methoxy and chloro being particularly preferred substituents. Good results are obtained when A is an unsubstituted azine group. Preferred azine groups are pyrazinyl, 3-pyridyl, 4-pyrimidinyl and 5-pyrimidinyl. In particular it is preferred that A represents a pyrazinyl group, most preferably an unsubstituted pyrazinyl group.

The alkyl substituents R and $R^1$ need not be the same, although this is usually preferred from a point of view of synthetic accessability. Preferably each of R and $R^1$ independently represents a methyl or an ethyl group, more preferably at least one of R and $R^1$ represents a methyl group and most preferably R and $R^1$ both represent methyl groups.

Acid addition salts, N-oxides and metal salt complexes of the compounds according to formula I are easily prepared, and essentially equal to these compounds — differing only in their "packaging". Therefore these salts, oxides and complexes are an embodiment of the present invention as well.

Thus, particularly preferred compounds according to Formula I include: N-(pyrazinylmethyl)-2,6-dimethylchloroacetanilide, N-(pyrazinylmethyl)-2-ethyl-6-methylchloroacetanilide, N-(pyrazinylmethyl)-2,6-diethylchloroacetanilide, N-(3-pyridylmethyl)-2,6-dimethylchloroacetanilide, N-(4-pyrimidinylmethyl)-2,6-dimethylchloroacetaniilide, N-(5-pyrimidinylmethyl)-2,6-dimethylchloroacetanilide and N-(4,6-dimethoxypyrimidin-5-ylmethyl)-2,6-dimethylchloroacetanilide, of which the first one is most preferred.

The invention also relates to a process for the preparation of a compound according to formula I, in which a compound of the general formula

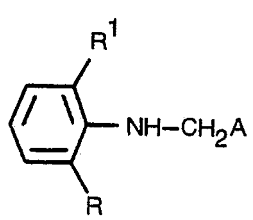

(II)

is acylated with a compound of the general formula

$$X{-}CO{-}CH_2Cl \qquad (III)$$

wherein A, R and $R^1$ have the meanings defined hereinabove, and X represents a suitable leaving group.

Suitable leaving groups X include carboxylates (the compounds of formula III being anhydrides), and — more suitably — halogen atoms, e.g. bromine or chlorine atoms (the compounds of formula III being acid halides). Suitably a diluting agent, e.g. a solvent, is present, as well as scavenging agent for the other reaction product, HX. Prefeably the leaving group X is a halogen atom, and the reaction is preferably carried out in the presence of an acid-binding agent, particularly a base.

When required, these compounds thus prepared are converted into the corresponding acid addition salt, N-oxides and metal salt complexes by methods well known in the art.

Compounds of general formula II are suitably prepared by the reaction of an aniline of formula

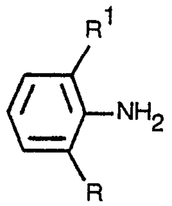

(IV)

with a heterocyclic methylchloride of formula (Cl-$CH_2$A, where R, $R^1$ and A have the meanings defined hereinbefore, preferably in the presence of an acid-binding agent and a diluting agent. The anilines of formula IV are generally known compounds, and often commercially obtainable. The heterocyclic methylchlorides can also be prepared by methods known per se.

The reactions mentioned hereinabove are normally carried out at temperatures between 0°C and 180°C, in particular between 20°C and 150°C, and at atmospheric pressure. Suitable acid-binding agents include all usual organic and inorganic acid-acceptors, e.g. alkali metal carbonates such as $Na_2CO_3$ and $K_2CO_3$, or tertiary amines, such as triethylamine. Suitable diluting agents include all usual inert organic solvents, e.g. dimethylformamide and toluene. The reactants are generally employed in substantially equimolar quantities, although it may be preferred sometimes to employ one of the reactants in excess to the other; for example an excess of the aniline compound of formula IV may be·used.

The invention also provides a herbicidal composition, which comprises a compound of formula I as defined above, together with at least one carrier, and a method of making such a composition which comprises bringing a compound of formula I into association with at least one carrier. The invention also provides the use as a herbicide of such a compound or composition according to the invention. Furthermore the invention relates to a method of combatting undesired plant growth, in particular undesired annual grass growth, at a locus by treating the locus with a compound or a composition according to the invention. The method may be used for the control of weeds in a wide variety of crops, for example, leguminous crops such as peas and beans, cotton, soyabean, plantation crops such as sugar cane or vines, and, especially, sugar beet and brassica crops, rape, mustard. Application to the locus may be pre-emergence or post-emergence. The dosage of active ingredient used may, for example, be from 0.05 to 4 kg/ha.

A carrier in a composition according to the invention is any material with which the active ingredient is formulated to facilitate application to the locus to be treated, or to facilitate storage, transport or handling. A carrier may be a solid or a liquid, including a material which is normally gaseous but which has been compressed to form a liquid, and any of the carriers normally used in formulating agricultural compositions may be used. Preferably compositions according to the invention contain 0.5 to 95% by weight of active ingredient.

Suitable solid carriers include natural and synthetic clays and silicates, for example natural silicas such as diatomaceous earths, magnesium silicate, for example talcs; magnesium aluminium silicates, for example attapulgites and vermiculites; aluminium silicates, for example kaolinites, montomorillonites and micas; calcium carbonate; calcium sulphate; ammonium sulphate; synthetic hydrated silicon oxides and synthetic calcium or aluminium silicates; elements, for example carbon and sulphur; natural and synthetic resins, for example coumarone resins, polyvinyl chloride, and styrene polymers and copolymers; solid polychlorophenols; bitumen; waxes, for example beeswax, paraffin wax, and chlorinated mineral waxes; and solid fertilisers, for example superphosphates.

Suitable liquid carriers include water; alcohols, for example isopropanol and glycols; ketones, for example acetone, methyl ethyl ketone, methyl isobutyl ketone and cyclohexanone; ethers; aromatic or araliphatic hydrocarbons, for example benzene, toluene and xylene; petroleum fractions, for example kerosine and light mineral oils; and chlorinated hydrocarbons, for example carbon tetrachloride, perchloroethylene and trichloroethane. Mixtures of different liquids are often suitable.

Agricultural compositions are often formulated and transported in a concentrated form which is subsequently diluted by the user before application. The presence of small amounts of carrier which is a surface-active agent facilitates this process of dilution. Thus preferably at least one carrier in a composition according to the invention is a surface-active agent. For example the composition may contain at least two carriers, at least one of which is a surface-active agent.

A surface-active agent may be an emulsifying agent, a dispersing agent or a wetting agent; it may be nonionic or ionic. Examples of suitable surface-active agents include the sodium or calcium salts of polyacrylic acids and lignin sulphonic acids; the condensation products of fatty acids or aliphatic amines or amides containing at least 12 carbon atoms in the molecule with ethylene oxide and/or propylene oxide; fatty acid esters of glycerol, sorbitan, sucrose or pentaerythritol; condensates of these with ethylene oxide and/or propylene oxide; condensation products of fatty alcohol or alkyl phenols, for example p-octylcresol, with ethylene oxide and/or propylene oxide; sulphates or sulphonates of these condensation products; alkali or alkaline earth metal salts, preferably sodium salts, of sulphuric or sulphonic acid esters containing at least 10 carbon atoms in the molecule, for example sodium lauryl sulphate, sodium secondary alkyl

sulphates, sodium salts of sulphonated castor oil, and sodium alkylaryl sulphonates such as sodium dodecylbenzene sulphonate; and polymers of ethylene oxide and copolymers of ethylene oxide and propylene oxide.

The compositions of the invention may for example be formulated as wettable powders, dusts, granules, solutions, emulsifiable concentrates, emulsions, suspension concentrates and aerosols. Wettable powders usually contain 25, 50 or 75% w of active ingredient and usually contain in addition to solid inert carrier, 3—10% w of stabiliser(s) and/or other additives such as penetrants or stickers. Dusts are usually formulated as a dust concentrate having a similar composition to that of a wettable powder but without a dispersant, and are diluted in the field with further solid carrier to give a composition usually containing ½—10% w of active ingredient. Granules are usually prepared to have a size between 10 and 100 BS mesh (1.676—0.152 mm), and may be manufactured by agglomeration or impregnation techniques. Generally, granules will contain ½—75%w active ingredient and 0—10% w of additives such as stabilisers, surfactants, slow release modifiers and binding agents. The so-called "dry flowable powders" consist of relatively small granules having a relatively high concentration of active ingredient. Emulsifiable concentrates usually contain, in addition to a solvent and, when necessary, co-solvent, 10—50% w/v active ingredient, 2—20% w/v emulsifiers and 0—20% w/v of other additives such as stabilisers, penetrants and corrosion inhibitors. Suspension concentrates are usually compounded so as to obtain a stable, non-sedimenting flowable product and usually contain 10—75% w active ingredient, 0.5—15% w of dispersing agents, 0.1—10% w of suspending agents such as protective colloids and thixotropic agents, 0—10% w of other additives such as defoamers, corrosion inhibitors, stabilisers, penetrants and stickers, and water or an organic liquid in which the active ingredient is substantially insoluble; certain organic solids or inorganic salts may be present dissolved in the formulation to assist in preventing sedimentation or as anti-freeze agents for water.

Aqueous dispersions and emulsions, for example compositions obtained by diluting a wettable powder or a concentrate according to the invention with water, also lie within the scope of the present invention. The said emulsions may be of the water-in-oil or of the oil-in-water type, and may have a thick 'mayonnaise'-like consistency.

The compositions of the invention may also contain other ingredients, for example, other compounds possessing herbicidal, insecticidal or fungicidal properties.

The following Examples illustrate the invention.

## Example 1

Preparation of N-(pyrazinylmethyl)-2,6-dimethylchloroacetanilide.

Chloroacetylchloride (8.17 g, 0.0723 mole) was added dropwise to a stirred solution of 14 g (0.0657 mole) N-(pyrazinylmethyl)-2,6-dimethylaniline, 5.71 g (0.0723 mole) dry pyridine and 82 ml dry tetrahydrofuran. The solution was kept in a conical flask in an ice/water bath, and the chloroacetylchloride was added at such a rate that the solution temperature was kept between 15 and 20°C. This reaction mixture was stirred at room temperature for 16 hours, with a stream of nitrogen bubbling through to evaporate off the tetrahydrofuran. A thin layer chromatogram (silica/ether) was taken of the starting materials and of the reaction mixture, after some time, which indicated that a reaction was taking place. The residue was then treated with water and extracted three times with ether. The combined ether extracts were then washed two times by water, and dried over $MgSO_4$. After filtering off the solid ($MgSO_4$), the ether was removed at room temperature. The resulting solid was recrystallised from cyclohexane, yielding 13.3 g (69.9% molar). The elemental analysis and the metling point are given in Table I.

The starting material N-(pyrazinylmethyl)-2,6-dimethyl-aniline had been prepared by selectively chlorinating 2-methylpyrazine using N-chlorosuccinimide and 2,2$^1$-azobis-2-methylpropionitrile, and then coupling the resulting 2-chloromethylpyrazine with 2,6-dimethylaniline, using an organic base (triethylamine). The yield based on 2-methylpyrazine was 65.1% molar.

## Examples 2—11

By methods analogous to those of Example 1, further compounds of the general formula I were prepared. Details are given in Table I.

TABLE I

| Example No. | in Formula I | | | Melting Point °C | Elemental Analysis | | | |
|---|---|---|---|---|---|---|---|---|
| | A | R | $R^1$ | | | C | H | N |
| 1 | pyrazinyl | $CH_3$ | $CH_3$ | 71—72 | Calc:<br>Found: | 62.2<br>62.1 | 5.5<br>5.4 | 14.5<br>14.3 |
| 2 | pyrazinyl | $CH_3$ | $C_2H_5$ | oil | Calc:<br>Found: | 63.2<br>62.9 | 6.0<br>6.1 | 13.85<br>13.8 |
| 3 | pyrazinyl | $C_2H_5$ | $C_2H_5$ | oil | Calc:<br>Found: | 64.2<br>63.2 | 6.3<br>6.3 | 13.3<br>12.9 |
| 4 | 2-pyridyl | $CH_3$ | $CH_3$ | oil | Calc:<br>Found: | 66.5<br>66.1 | 5.9<br>6.0 | 9.7<br>9.0 |
| 5 | 3-pyridyl | $CH_3$ | $CH_3$ | 125 | Calc:<br>Found: | 66.5<br>66.1 | 5.9<br>6.0 | 9.7<br>9.6 |
| 6 | 3-pyridyl | $C_2H_5$ | $CH_3$ | oil | Calc:<br>Found: | 67.9<br>67.2 | 6.3<br>6.4 | 9.3<br>9.1 |
| 7 | 4-pyridyl | $CH_3$ | $CH_3$ | 118 | Calc:<br>Found: | 66.5<br>66.2 | 5.9<br>6.0 | 9.7<br>9.6 |
| 8 | 4-pyrimidinyl | $CH_3$ | $CH_3$ | oil | Calc:<br>Found: | 62.2<br>62.5 | 5.5<br>5.7 | 14.5<br>14.4 |
| 9 | 5-pyrimidinyl | $CH_3$ | $CH_3$ | oil | Calc:<br>Found: | 62.2<br>62.3 | 5.5<br>5.6 | 14.5<br>13.9 |
| 10 | 5-pyrimidinyl-4,6-dichloro | $CH_3$ | $CH_3$ | 158—160 | Calc:<br>Found: | 50.2<br>50.2 | 3.9<br>3.9 | 11.7<br>11.6 |
| 11 | 5-pyrimidinyl-4,6-dimethoxy | $CH_3$ | $CH_3$ | 111—112 | Calc:<br>Found: | 58.4<br>58.3 | 5.7<br>5.8 | 12.0<br>11.9 |

Example 12

Herbicidal Activity (General)

To evaluate their herbicidal activity, compounds according to the invention were tested using as a representative range of plants: maize, *Zea mays* (Mz); rice, *Oryza sativa* (R); barnyard grass, *Echinochloa crusgalli* (BG); oat, *Avena sativa* (O); linseed, *Linum usitatissisum* (L); mustard, *Sinapis alba* (M); sugar beet, *Beta vulgaris* (SB) and soya bean, *Glycine max* (S).

The tests fall into two categories, pre-emergence and post-emergence. The pre-emergence tests involved spraying a liquid formulation of the compound onto the soil in which the seeds of the plant species mentioned above had recently been sown. The post-emergence tests involved two types of test, viz., soil drench and foliar spray tests. In the soil drench tests the soil in which the seedling plants of the above species were growing was drenched with a liquid formulation containing a compound of the invention, and in the foliar spray tests the seedling plants were sprayed with such a formulation.

The soil used in the tests was a prepared horticultural loam.

The formulations used in the tests were prepared by diluting with water, solutions of the test compounds in acetone containing 0.4% by weight of an alkylphenol/ethylene oxide condensate available under the trade mark TRITON X-155. The acetone solutions were diluted with water and the resulting formulations applied at dosage levels corresponding to 5 kg and/or 1 lg of active material per hectare in a volume equivalent to 600 litres per hectare in the soil spray and foliar spray tests, and at a dosage level equivalent to 10 kilograms of active material per hectare in a volume equivalent to approximately 3,000 litres per hectare in the soil drench tests.

In the pre-emergence tests untreated sown soil and in the post-emergence tests untreated soil bearing seedling plants were used as controls.

The herbicidal effects of the test compounds were assessed visually twelve days after spraying the foliage and the soil, and thirteen days after drenching the soil and were recorded on a 0—9 scale. A rating 0 indicates growth as untreated control, a rating 9 indicates death. An increase of 1 unit on the linear scale approximates to a 10% increase in the level of effect.

The results of the tests are set out in Table II below.

TABLE II

| Compound of Example No. | Soil Drench 10 kg/ha | | | | | | | | Dosage kg/ha | Foliar Spray | | | | | | | | Pre-emergence | | | | | | | |
|---|---|---|---|---|---|---|---|---|---|---|---|---|---|---|---|---|---|---|---|---|---|---|---|---|---|
| | Mz | R | BG | O | L | M | SB | S | | Mz | R | BG | O | L | M | SB | S | Mz | R | BG | O | L | M | SB | S |
| 1 | 7 | 7 | 8 | 7 | 6 | 6 | 5 | 4 | 5 | 6 | 7 | 8 | 6 | 4 | 5 | 4 | 6 | 6 | 8 | 9 | 7 | 5 | 6 | 7 | 5 |
| | | | | | | | | | 1 | 3 | 5 | 7 | 3 | 3 | 3 | 2 | 5 | 4 | 7 | 8 | 6 | 2 | 5 | 4 | 0 |
| 2 | 6 | 7 | 8 | 7 | 4 | 4 | 2 | 3 | 5 | 5 | 5 | 7 | 5 | 4 | 4 | 2 | 5 | 6 | 9 | 9 | 6 | 2 | 4 | 5 | 4 |
| | | | | | | | | | 1 | 3 | 4 | 6 | 2 | 0 | 4 | 0 | 4 | 0 | 4 | 8 | 4 | 0 | 2 | 0 | 3 |
| 3 | 5 | 6 | 8 | 7 | 0 | 3 | 0 | 0 | 5 | 4 | 6 | 7 | 5 | 4 | 2 | 2 | 4 | 3 | 8 | 9 | 7 | 4 | 5 | 6 | 5 |
| | | | | | | | | | 1 | 0 | 0 | 4 | 0 | 0 | 0 | 0 | 4 | 0 | 4 | 8 | 0 | 0 | 0 | 4 | 0 |
| 5 | 6 | 7 | 7 | 6 | 0 | 0 | 0 | 0 | 5 | 0 | 5 | 7 | 0 | 0 | 0 | 0 | 4 | 0 | 5 | 8 | 6 | 0 | 0 | 4 | 0 |
| | | | | | | | | | 1 | 0 | 0 | 3 | 0 | 0 | 0 | 0 | 3 | 0 | 0 | 7 | 4 | 0 | 0 | 4 | 0 |
| 6 | 2 | 7 | 6 | 6 | 0 | 0 | 0 | 0 | 5 | 0 | 0 | 2 | 0 | 0 | 0 | 0 | 0 | 0 | 0 | 7 | 0 | 0 | 0 | 0 | 2 |
| | | | | | | | | | 1 | 0 | 0 | 0 | 0 | 0 | 0 | 0 | 0 | 0 | 0 | 2 | 0 | 0 | 0 | 0 | 2 |
| 8 | 7 | 7 | 7 | 7 | 5 | 5 | 2 | 3 | 5 | 5 | 6 | 7 | 6 | 5 | 0 | 0 | 4 | 6 | 8 | 8 | 5 | 0 | 0 | 0 | 4 |
| | | | | | | | | | 1 | 0 | 4 | 7 | 2 | 2 | 0 | 0 | 3 | 3 | 7 | 7 | 4 | 0 | 0 | 0 | 3 |
| 9 | 7 | 7 | 7 | 7 | 2 | 2 | 6 | 3 | 5 | 3 | 6 | 6 | 0 | 0 | 0 | 0 | 4 | 5 | 6 | 8 | 5 | 0 | 0 | 0 | 2 |
| | | | | | | | | | 1 | 1 | 6 | 6 | 0 | 0 | 0 | 0 | 4 | 0 | 4 | 7 | 2 | 0 | 0 | 0 | 0 |
| 11 | 7 | 5 | 6 | 3 | 0 | 0 | 0 | 0 | 5 | 6 | 4 | 5 | 0 | 2 | 3 | 3 | 4 | 0 | 4 | 8 | 3 | 0 | 3 | 0 | 0 |
| | | | | | | | | | 1 | 0 | 0 | 4 | 0 | 0 | 0 | 0 | 3 | 0 | 3 | 7 | 0 | 0 | 2 | 0 | 0 |

Example 13

Herbicidal Activity (Selective)

The compounds of Examples 1 and 2 were compared with two known compounds, prynachlor and alachlor, to illustrate the selective herbicidal properties of the compounds according to the invention.

Prynachlor is a chloroacetanilide compound according to formula I, wherein $R=R^1=CH_3$ and A=1-pyrazolyl; alachlor is the compound according to formula I, wherein $R=R^1$=ethyl and A=methoxy.

The following plants were subjected to a pre-emergence spray test as in Example 12; sugar beet, *Beta vulgaris* (SB); oil-seed rape, *Brassica napus* (RA); barnyard grass, *Echinocloa crusgalli* (BG); blackgrass, *Alopecurus myosuroides* (AM); wild oat, *Avena fatua/ludoviciana* (WO); and ryegrass, *Lolium multiflorum* (RG). All compounds were applied at doses of 3.0, 1.0, 0.3 amd 0.1 kg/ha to all species, in duplicate, as a single dose pre-emergence spray in a total volume of 600 litres/ha. The phytotoxicity was assessed using the standard 0—9 scale described in Example 12, at 12, 19 and 26 days after treatment. On the data thus obtained a probit analysis was carried out to give $GID_{50}$s and $_{90}$s, that is, the doses required to give a 50% or 90% level of effect. The GID values are presented in tables III, IV and V. GID values above 100, corresponding to a very high tolerance of the species for the compound tested, are shown as 99.99.

From these results, in particular from the $GID_{90}$s, it can be seen that all four compounds tested are very toxic against weeds, but that alachlor and prynachlor do affect the rape and especially the sugar beet at the dosages required to combat the weeds, especially the wild oat and the rye grass.

TABLE III

| Species | GID values, assessment after 12 days | | | | | | | |
|---|---|---|---|---|---|---|---|---|
| | Ex. 1 | | Ex. 2 | | prynachlor | | alachlor | |
| | 50 | 90 | 50 | 90 | 50 | 90 | 50 | 90 |
| SB | 59.02 | 99.99 | 15.56 | 99.99 | 0.06 | 2.78 | 0.17 | 7.32 |
| RA | 99.99 | 99.99 | 99.99 | 99.99 | 1.82 | 15.64 | 6.03 | 51.86 |
| BG | 0.07 | 0.22 | 0.22 | 0.75 | 0.05 | 0.17 | 0.06 | 0.21 |
| AM | 4.43 | 20.59 | 2.33 | 10.84 | 0.17 | 0.77 | 1.66 | 7.72 |
| WO | 2.72 | 12.84 | 4.47 | 21.12 | 0.59 | 2.78 | 1.57 | 7.43 |
| RG | 1.46 | 6.67 | 1.90 | 8.70 | 0.18 | 0.83 | 0.93 | 4.24 |

TABLE IV

| Species | GID values, assessment after 19 days | | | | | | | |
|---|---|---|---|---|---|---|---|---|
| | Ex. 1 | | Ex. 2 | | prynachlor | | alachlor | |
| | 50 | 90 | 50 | 90 | 50 | 90 | 50 | 90 |
| SB | 3.23 | 99.99 | 2.82 | 99.99 | 0.05 | 2.26 | 0.28 | 12.50 |
| RA | 99.99 | 99.99 | 5.58 | 80.25 | 3.37 | 48.54 | 14.79 | 99.99 |
| BG | 0.07 | 0.18 | 0.28 | 0.73 | 0.00 | 0.00 | 0.07 | 0.18 |
| AM | 3.03 | 25.19 | 1.09 | 9.06 | 0.05 | 0.39 | 0.78 | 6.45 |
| WO | 2.03 | 9.38 | 2.81 | 12.96 | 0.34 | 1.58 | 1.26 | 5.80 |
| RG | 0.50 | 2.31 | 0.83 | 3.83 | 0.08 | 0.35 | 0.48 | 2.22 |

TABLE V

| Species | GID values, assessment after 26 days | | | | | | | |
|---|---|---|---|---|---|---|---|---|
| | Ex. 1 | | Ex. 2 | | prynachlor | | alachlor | |
| | 50 | 90 | 50 | 90 | 50 | 90 | 50 | 90 |
| SB | 15.05 | 99.99 | 17.60 | 99.99 | 0.18 | 2.52 | 0.84 | 11.57 |
| RA | 99.99 | 99.99 | 99.99 | 99.99 | 1.84 | 7.67 | 12.69 | 52.78 |
| BG | 0.07 | 0.20 | 0.25 | 0.76 | 0.00 | 0.00 | 0.06 | 0.19 |
| AM | 1.96 | 15.45 | 1.52 | 11.95 | 0.03 | 0.24 | 0.64 | 5.06 |
| WO | 2.43 | 10.56 | 3.23 | 14.01 | 0.32 | 1.39 | 1.34 | 5.83 |
| RG | 0.65 | 2.09 | 1.04 | 3.33 | 0.10 | 0.31 | 0.93 | 2.97 |

**Claims for the Contracting States: BE CH DE FR GB IT LI LU NL SE**

1. Chloroacetanilide derivatives of the general formula I:

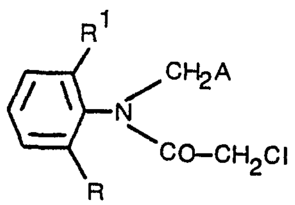

(I)

wherein A represents an optionally substituted azine selected from pyridyl, pyrazinyl, pyridazinyl, 4-pyrimidinyl and 5-pyrimidinyl, optional substitutents being selected from $C_{1-4}$ alkyl, $C_{1-4}$ alkoxy, $C_{1-4}$ haloalkyl and hydroxy groups and halogen atoms; and R and $R^1$, which may be the same or different, each independently represents an alkyl group having up to four carbon atoms.

2. A compound as claimed in claim 1, wherein the azine group A is unsubstituted, or is substituted by one or more moieties selected from methyl, ethyl, methoxy, propoxy, trifluoromethyl and chloromethyl groups, and chloro and bromo atoms.

3. A compound as claimed in claim 1 or 2, wherein the azine group A is a pyrazinyl, 3-pyridyl, 4-pyrimidinyl or 5-pyrimidinyl group.

4. A compound as claimed in any one of claims 1 to 3, wherein each of R and $R^1$ independently represents a methyl or an ethyl group.

5. A compound as claimed in claim 1, wherein R and $R^1$ each represents a methyl group and A represents a pyrazinyl, 2-, 3-, or 4-pyridyl, 4- or 5-pyrimidinyl, or 5-pyrimidinyl, 4,6-dichloro or 5-pyrimidinyl, 4,6-dimethoxy group; or R and $R^1$ each represents an ethyl group and A represents a pyrazinyl group; or one of R and $R^1$ represents a methyl group, the other represents an ethyl group and A represents a pyrazinyl group.

6. N-(pyrazinylmethyl)-2,6-dimethylchloroacetanilide.

7. Process for the preparation of a compound as claimed in claim 1, in which a compound of the general formula

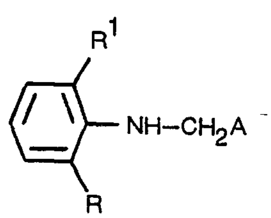

(II)

is acylated with a compound of the general formula

$$X{-}CO{-}CH_2Cl \qquad (III)$$

wherein A, R and $R^1$ have the meanings defined in claim 1, and X represents a suitable leaving group.

8. A process as claimed in claim 7, wherein the leaving group is a halogen atom, and the reaction is carried out in the presence of an acid-binding agent.

9. Herbicidal composition, which comprises a compound as claimed in any one of claims 1 to 6, together with a carrier.

10. A composition as claimed in claim 9, which comprises at least two carriers, at least one of which is a surface-active agent.

11. Method of combatting undesired plant growth at a locus, which comprises treating the locus with a compound as claimed in any one of claims 1 to 6, or a composition as claimed in either of claims 9 or 10.

**Claims for the Contracting State: AT**

1. A herbicidal composition which comprises an active ingredient and a carrier, characterized in that the active ingredient is a chloroacetanilide derivative of the general formula I:

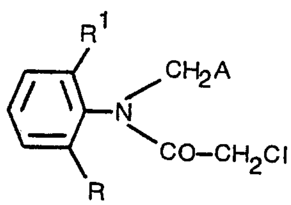

(I)

wherein A represents an optionally substituted azine selected from pyridyl, pyrazinyl, pyridazinyl, 4-pyrimidinyl and 5-pyrimidinyl, optional substitutents being selected from $C_{1-4}$ alkyl, $C_{1-4}$ alkoxy, $C_{1-4}$ haloalkyl and hydroxy groups and halogen atoms; and R and $R^1$, which may be the same or different, each independently represents an alkyl group having up to four carbon atoms.

2. A composition as claimed in claim 1, wherein the azine group A is unsubstituted, or is substituted by one or more moieties selected from methyl, ethyl, methoxy, propoxy, trifluoromethyl and chloromethyl groups, and chloro and bromo atoms.

3. A composition as claimed in claim 1 or 2, wherein the azine group A is a pyrazinyl, 3-pyridyl, 4-pyrimidinyl or 5-pyrimidinyl group.

4. A composition as claimed in any one of claims 1 to 3, wherein each of R and $R^1$ independently represents a methyl or an ethyl group.

5. A composition as claimed in claim 1, wherein R and $R^1$ each represents a methyl group and A represents a pyrazinyl, 2-, 3-, or 4-pyridyl, 4- or 5-pyrimidinyl, or 5-pyrimidinyl, 4,6-dichloro or 5-pyrimidinyl, 4,6-dimethoxy group; or R and $R^1$ each represents an ethyl group and A represents a pyrazinyl group; or one of R and $R^1$ represents a methyl group, the other represents an ethyl group and A represents a pyrazinyl group.

6. A composition as claimed in claim 1, containing N-(pyrazinylmethyl)-2,6-dimethylchloroacetanilide as active ingredient.

7. A composition as claimed in any preceding claim, which comprises at least two carriers, at least one of which is a surface-active agent.

8. Process for the preparation of a compound as claimed in claim 1, in which a compound of the general formula

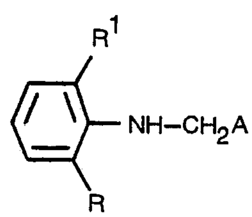

(II)

is acylated with a compound of the general formula

$$X—CO—CH_2Cl \quad (III)$$

wherein A, R and $R^1$ have the meanings defined in claim 1, and X represents a suitable leaving group.

9. A process as claimed in claim 8, wherein the leaving group is a halogen atom, and the reaction is carried out in the presence of an acid-binding agent.

10. Method of combatting undesired plant growth at a locus, which comprises treating the locus with a compound as defined in any one of claims 1 to 6.

**Patentansprüche für die Vertragsstaaten: BE CH DE FR GB IT LI LU NL SE**

1. Chloroacetanilidderivative der allgemeinen Formel I:

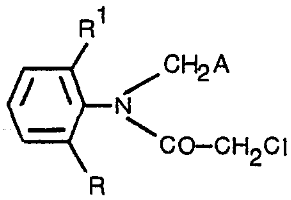

(I)

worin A ein gegebenenfalls substituiertes Azin, ausgewählt unter Pyridyl, Pyrazinyl, Pyridazinyl, 4-Pyrimidinyl und 5-Pyrimidinyl, darstellt und die fakultativen Substituenten unter $C_{1-4}$-Alkylgruppen, $C_{1-4}$-Alkoxygruppen, $C_{1-4}$-Halogenalkylgruppen und Hydroxygruppen und Halogenatomen ausgewählt sind; und R und $R^1$, die gleich oder verschieden sein können, jeweils unabhängig voneinander eine Alkylgruppe mit bis zu 4 Kohlenstoffatomen bedeuten.

2. Verbindung nach Anspruch 1, worin die Azingruppe A unsubstituiert oder durch einen oder mehrere Reste, ausgewählt unter Methyl-, Ethyl-, Methoxy-, Propoxy-, Trifluormethyl- und Chlormethylgruppen und Chlor- und Bromatomen, substituiert ist.

3. Verbindung nach Anspruch 1 oder 2, worin die Azingruppe A eine Pyrazinyl-, 3-Pyridyl-, 4-Pyrimidinyl- oder 5-Pyrimidinylgruppe ist.

4. Verbindung nach einem der Ansprüche 1—3, worin jeder der Reste R und $R^1$ unabhängig voneinander eine Methyl- oder eine Ethylgruppe bedeutet.

5. Verbindung nach Anspruch 1, worin R und $R^1$ jeweils eine Methylgruppe darstellen und A eine Pyrazinyl-Gruppe, 2-, 3- oder 4-Pyridylgruppe, 4- oder 5-Pyrimidinylgruppe oder 5-Pyrimidinyl-4,6-dichlorgruppe oder 5-Pyrimidinyl-4,6-dimethoxygruppe bedeutet; oder R und $R^1$ jeweils eine Ethylgruppe darstellen und A eine Pyrazinylgruppe bedeutet; oder einer der Reste R und $R^1$ eine Methylgruppe darstellt und der andere eine Ethylgruppe bedeutet und A eine Pyrazinylgruppe darstellt.

6. N-(Pyrazinylmethyl)-2,6-dimethylchloracetanilid.

7. Verfahren zur Herstellung einer Verbindung nach Anspruch 1, worin eine Verbindung der allgemeinen Formel

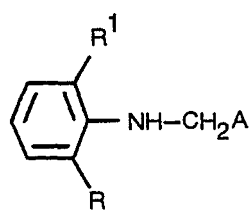

(II)

mit einer Verbindung der allgemeinen Formel

$$X{-}CO{-}CH_2Cl \qquad (III)$$

acyliert wird, in welchen Formeln A, R und $R^1$ die in Anspruch 1 definierten Bedeutungen aufweisen und X eine geeignete Leaving-Gruppe darstellt.

8. Verfahren nach Anspruch 7, worin die Leaving-Gruppe ein Halogenatom ist und die Reaktion in Gegenwart eines säurebindenden Mittels ausgeführt wird.

9. Herbizide Zusammensetzung, welche eine Verbindung, wie in einen der Ansprucñche 1 bis 6 beansprucht, zusammen mit einem Träger umfaßt.

10. Zusammensetzung nach Anspruch 9, welche wenigstens zwei Träger umfaßt, von denen wenigstens einer ein oberflächenaktives Mittel ist.

11. Verfahren zur Bekämpfung von unerwünschtem Pflanzenwuchs an einem Ort, welches ein Behandeln des Ortes mit einer Verbindung, wie in einem der Ansprüche 1 bis 6 beansprucht, oder mit einer Zusammensetzung, wie in Anspruch 9 oder 10 beansprucht, umfaßt.

**Patentansprüche für den Vertragsstaat: AT**

1. Herbizide Zusammensetzung, welche einen aktiven Bestandteil und einen Träger umfaßt, dadurch gekennzeichnet, daß der aktive Bestandteil ein Chloracetanilidderivat der allgemeinen, Formel I:

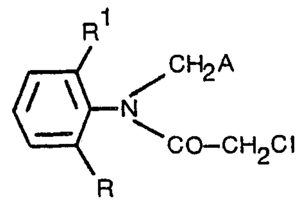

(I)

ist, worin A ein gegebenenfalls substituiertes Azin, ausgewählt unter Pyridyl, Pyrazinyl, Pyridazinyl, 4-Pyrimidinyl und 5-Pyrimidinyl, darstellt und die fakultativen Substituenten unter $C_{1-4}$-Alkylgruppen, $C_{1-4}$-Alkoxygruppen, $C_{1-4}$-Halogenalkylgruppen und Hydroxygruppen und Halogenatomen ausgewählt sind; und R und $R^1$, die gleich oder verschieden sein können, jeweils unabhängig voneinander eine Alkylgruppe mit bis zu 4 Kohlenstoffatomen bedeuten.

2. Zusammensetzung nach Anspruch 1, worin die Azingruppe A unsubstituiert oder durch einen oder mehrere Reste, ausgewählt unter Methyl-, Ethyl-, Methoxy-, Propoxy-, Trifluormethyl- und Chlormethylgruppen und Chlor- und Bromatomen, substituiert ist.

3. Zusammensetzung nach Anspruch 1 oder 2, worin die Azingruppe A eine Pyrazinyl-, 3-Pyridyl-, 4-Pyrimidinyl- oder 5-Pyrimidinylgruppe ist.

4. Zusammensetzung nach einem der Ansprüche 1—3, worin jeder der Reste R und R undabhängig voneinander eine Methyl- oder eine Ethylgruppe bedeutet.

5. Zusammensetzung nach Anspruch 1, worin R und $R^1$ jeweils eine Methylgruppe darstellen und A eine Pyrazinyl-Gruppe, 2-, 3- oder 4-Pyridylgruppe, 4- oder 5-Pyrimidinylgruppe oder 5-Pyrimidinyl-4,6-dichlorgruppe oder 5-Pyrimidinyl-4,6-dimethoxygruppe bedeutet; oder R und $R^1$ jeweils eine Ethylgruppe darstellen und A eine Pyrazinylgruppe bedeutet; oder einer der Reste R und $R^1$ eine Methylgruppe darstellt und der andere eine Ethylgruppe bedeutet und A eine Pyrazinylgruppe darstellt.

6. Zusammensetzung nach Anspruch 1, enthaltend N-(Pyrazinylmethyl)-2,6-dimethylchloracetanilid als wirksamen Bestandteil.

7. Zusammensetzung nach einem der vorstehenden Ansprüche, welche wenigstens 2 Träger umfaßt, von denen wenigstens einer ein oberflächenaktives Mittel ist.

8. Verfahren zur Herstellung einer Verbindung nach Anspruch 1, worin eine Verbindung der allgemeinen Formel

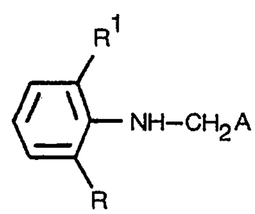

(II)

mit einer Verbindung der allgemeinen Formel

$$X—CO—CH_2Cl \quad (III)$$

acyliert wird, in welchen Formeln A, R und $R^1$ die in Anspruch 1 definierten Bedeutungen aufweisen und X eine geeignete Leaving-Gruppe darstellt.

9. Verfahren nach Anspruch 8, worin die Leaving-Gruppe ein Halogenatom ist und die Reaktion in Gegenwart eines säurebindenden Mittels ausgeführt wird.

10. Verfahren zur Bekämpfung von unerwünschtem Pflanzenwuchs an einem Ort, welches ein Behandeln des Ortes mit einer Verbindung, wie in einem der Ansprüche 1 bis 6 definiert, umfaßt.

**Revendications pour les Etats contractants: BE CH LI DE FR GB IT LU NL SE**

1. Dérivés de chloroacétanilide de la formule générale I:

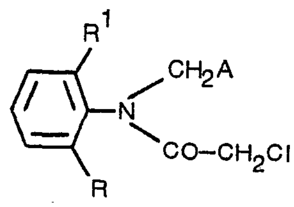

(I)

où A représente un groupe azine éventuellement substitué choisi parmi les groupes pyridyle, pyrazinyle, pyridazinyle, 4-pyrimidinyle et 5-pyrimidinyle, les substituants éventuels étant choisis parmi des groupes alcoyle en $C_{1-4}$, alcoxy en $C_{1-4}$, haloalcoyle en $C_{1-4}$ et hydroxy et des atomes d'halogènes; et R et $R^1$, qui peuvent être identiques ou différents, représentent chacun indépendamment un groupe alcoyle ayant jusqu'à quatre atomes de carbone.

2. Un composé selon la revendication 1, dans lequel le groupe azine A est non-substitué, ou est substitué par une ou deux portions choisies parmi les groupes méthyle, éthyle, méthoxy, propoxy, trifluorométhyle et chlorométhyle et les atomes de chlore et de brome.

3. Un composé selon la revendication 1 ou 2, dans lequel le groupe azine A est une groupe pyrazinyle, 3-pyridyle, 4-pyrimidinyle ou 5-pyrimidinyle.

4. Un composé selon l'une quelconque des revendications 1 à 3, dans lequel R et $R^1$ représentent chacun indépendamment un groupe méthyle ou éthyle.

5. Un composé selon la revendication 1, dans lequel R et $R^1$ représentent chacun un groupe méthyle et A représente un groupe pyrazinyle, 2-, 3- ou 4-pyridyle, 4- ou 5-pyrimidinyle ou 5-pyrimidinyl, 4,6-dichloro ou 5-pyrimidinyl, 4,6-diméthoxy; ou R et $R^1$ représentent chacun un groupe éthyle et A représente un groupe pyrazinyle; ou un de R et $R^1$ représente un groupe méthyle, l'autre représente un groupe éthyle et A représente un groupe pyrazinyle.

6. Le N-(pyrazinylméthyl-2,6-diméthylchloroacétanilide.

7. Procédé de préparation d'un composé tel que revendiqué dans la revendication 1, selon lequel un composé de la formule générale

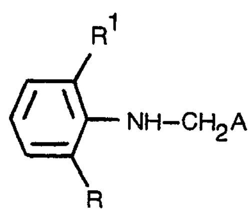

(II)

est acylé avec un composé de la formule générale

$$X—CO—CH_2Cl \qquad (III)$$

où A, R et $R^1$ ont les significations définies dans la revendication 1 et X représente un groupe sortant approprié.

8. Un procédé selon la revendication 7, dans lequel le groupe sortant est un atome d'halogène et la réaction est conduite en présence d'un agent fixant les acides.

9. Composition herbicide, qui comprend un composé selon l'une quelconque des revendications 1 à 6 en même temps qu'un véhicule.

10. Une composition selon la revendication 9, qui comprend au moins deux véhicules, dont au moins un est un agent tensio-actif.

11. Méthode pour combattre la croissance de plantes indésirables en un lieu, qui comprend le traitement du lieu par un composé selon l'une quelconque des revendications 1 à 6 ou une composition selon l'une des revendications 9 ou 10.

**Revendications pour l'Etat contractant: AT**

1. Une composition herbicide qui comprend un ingrédient actif et un véhicule, caractérisée en ce que l'ingrédient actif est un dérivé de chloroacétanilide de la formule générale I:

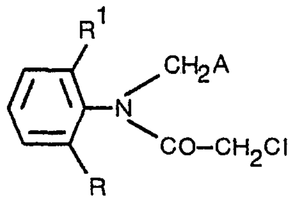

(I)

où A représente un groupe azine éventuellement substitué choisi parmi les groupes pyridyle, pyrazinyle, pyridazinyle, 4-pyrimidinyle et 5-pyrimidinyle, les substituants éventuels étant choisis parmi des groupes alcoyle en $C_{1-4}$, alcoxy en $C_{1-4}$, haloalcoyle en $C_{1-4}$ et hydroxy et des atomes d'halogènes; et R et $R^1$, qui peuvent être identiques ou différents, représentent chacun indépendamment un groupe alcoyle ayant jusqu'à quatre atomes de carbone.

2. Une composition selon la revendication 1, dans lequel le groupe azine A est non-substitué, ou est substitué par une ou deux portions choisies parmi les groupes méthyle, éthyle, méthoxy, propoxy, trifluorométhyle et chlorométhyle et les atomes de chlore et de brome.

3. Une composition selon la revendication 1 ou 2, dans lequel le groupe azine A est un groupe pyrazinyle, 3-pyridyle, 4-pyrimidinyle ou 5-pyrimidinyle.

4. Une composition selon l'une quelconque des revendications 1 à 3, dans lequel R et $R^1$ représentent chacun indépendamment un groupe méthyle ou éthyle.

5. Une composition selon la revendication 1, dans lequel R et $R^1$ représentent chacun un groupe méthyle et A représente un groupe pyrazinyle, 2-, 3- ou 4-pyridyle, 4- ou 5-pyrimidinyle ou 5-pyrimidinyl, 4,6-dichloro ou 5-pyrimidinyl, 4,6-diméthoxy; ou R et $R^1$ représentent chacun un groupe éthyle et A représente un groupe pyrazinyle; ou un de R et $R^1$ représente un groupe méthyle, l'autre représente un groupe éthyle et A représente un groupe pyrazinyle.

6. Une composition selon la revendication 1, contenant du N-(pyrazinylméthyl-2,6-diméthylchloroacétanilide comme ingrédient actif.

7. Une composition selon l'une quelconque des revendications précédentes, qui comprend au moins deux véhicules, dont au moins un est un agent tensio-actif.

8. Procédé pour la préparation d'un composé tel que défini dans la revendication 1, selon lequel un composé de la formule générale

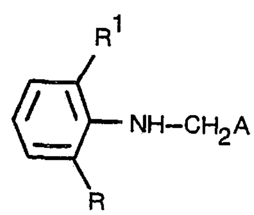

(II)

est acylé avec un composé de la formule générale

$$X—CO—CH_2Cl \qquad (III)$$

où A, R et $R^1$ ont les significations définies dans la revendication 1 et X représente un groupe sortant approprié.

9. Un procédé selon la revendication 8, dans lequel le groupe sortant est un atome d'halogène et la réaction est conduite en présence d'un agent fixant les acides.

10. Méthode pour combattre la croissance de plantes indésirables en un lieu, qui comprend le traitement du lieu par un composé tel que défini dans l'une quelconque des revendications 1 à 6.